(19) Europäisches Patentamt
European Patent Office
Office européen des brevets

(11) Veröffentlichungsnummer: **0 417 637 A2**

# (12) EUROPÄISCHE PATENTANMELDUNG

(21) Anmeldenummer: **90117147.0**

(51) Int. Cl.5: **A61K 31/425**, A61K 31/55

(22) Anmeldetag: **06.09.90**

(30) Priorität: **11.09.89 DE 3930282**

(43) Veröffentlichungstag der Anmeldung:
**20.03.91 Patentblatt 91/12**

(84) Benannte Vertragsstaaten:
**AT BE CH DE DK ES FR GB GR IT LI LU NL SE**

(71) Anmelder: **BOEHRINGER INGELHEIM KG**
**Postfach 200**
**W-6507 Ingelheim am Rhein(DE)**
(84) **BE CH DE DK ES FR GR IT LI LU NL SE AT**

Anmelder: **BOEHRINGER INGELHEIM INTERNATIONAL GmbH**
**Postfach 20**
**W-6507 Ingelheim am Rhein(DE)**
(84) **GB**

(72) Erfinder: **Kutter, Eberhard, Prof. Dr.**
**Eisenacher Strasse 19**
**W-6507 Ingelheim am Rhein(DE)**
Erfinder: **Schingnitz, Günter, Dr.**
**Unter den Gärten 10**
**W-6550 Bad Kreuznach(DE)**

## (54) Verwendung von Dopamin-Autorezeptor-Agonisten bei der Behandlung von Drogenabhängigkeit.

(57) Gegenstand der Erfindung ist die Verwendung von Dopamin-Autorezeptor-Agonisten, insbesondere von 6-Allyl-2-amino-5,6,7,8-tetrahydro-4H-thiazolo[4,5-d]-azepin (BHT 920) und (S)-2-Amino-4,5,6,7-tetrahydro-6-n-propylamino-benzo-thiazol (SND 919), für die Herstellung von Medikamenten für die Behandlung von Drogenabhängigkeit.

EP 0 417 637 A2

# VERWENDUNG VON DOPAMIN-AUTOREZEPTOR-AGONISTEN BEI DER BEHANDLUNG VON DROGENABHÄNGIGKEIT

Gegenstand der Erfindung ist die Verwendung von Dopamin-Autorezeptor-Agonisten, insbesondere von 6-Allyl-2-amino-5,6,7,8-tetrahydro-4H-thiazolo[4,5-d]azepin (BHT 920) und (S)-2-Amino-4,5,6,7-tetrahydro-6-n-propylamino-benzothiazol, für die Herstellung von Medikamenten für die Behandlung von Drogenabhängigkeit.

In den belgischen Patentschriften 684 415 und 771 330 werden u.a. Thiazolo- und Oxazolo-Derivate der allgemeinen Formel

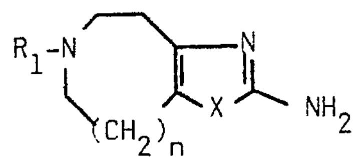

in der

$R_1$ ein Wasserstoffatom, eine gegebenenfalls durch eine Hydroxylgruppe substituierte Alkylgruppe mit 1 bis 4 Kohlenstoffatomen, eine gegebenenfalls durch ein Halogenatom, eine Methyl- oder Methoxygruppe substituierte Benzylgruppe oder eine Allylgruppe,

n die Zahl 2 oder auch 1, wenn X ein Schwefelatom darstellt, und

X ein Sauerstoff- oder Schwefelatom bedeutet, und deren physiologisch verträgliche Säureadditionssalze mit anorganischen oder organischen Säuren beschrieben.

Aus den belgischen patentschriften 684 415 und 771 330 ist bekannt, daß die Verbindungen der allgemeinen Formel I und deren physiologisch verträgliche Säureadditionssalze wertvolle pharmakologische Eigenschaften aufweisen. So weisen die in der belgischen Patentschrift 684 415 beschriebenen Verbindungen insbesondere eine analgetische, sedative, antitussive, antipyretische und antiphlogistische Wirkung und die in der belgischen Patentschrift 771 330 beschriebenen Verbindungen je nach ihrer Substitution insbesondere eine blutdrucksenkende, sedative, hustenstillende und/oder antiphlogistische Wirkung auf. Desweiteren geht aus den obengenannten Patentschriften hervor, daß die Thiazolo-Derivate der allgemeinen Formel I, in der $R_1$ eine Alkylgruppe mit 1 bis 4 Kohlenstoffatomen oder eine Allylgruppe, n die Zahl 2 und X ein Schwefelatom bedeuten, insbesondere eine blutdrucksenkende Wirkung und die Oxazolo-Derivate der allgemeinen Formel I, in der $R_1$ ein Wasserstoffatom, eine gegebenenfalls durch eine Hydroxygruppe substituierte Alkylgruppe mit 1 bis 4 Kohlenstoffatomen oder eine Allylgruppe, n die Zahl 2 und X ein Sauerstoffatom bedeuten, insbesondere hustenstillende Eigenschaften aufweisen.

Es ist aus der EP-Al-0 005 732 bekannt, daß die Verbindungen der obigen allgemeinen Formel I, in der n die Zahl 2 darstellt, auch eine antanginöse Wirksamkeit aufweisen.

Ferner ist aus dem US-Patent Nr. 4 400 378 bekannt, daß die Verbindungen der allgemeinen Formel I auch eine Antiglaucoma-Wirksamkeit aufweisen.

Gemäß den bisher vorliegenden wissenschaftlichen Veröffentlichungen wurde die Verbindung 2-Amino-6-allyl-5,6,7,8-tetrahydro-4H-thiazolo[4,5-d]azepin dihydrochlorid (BHT 920) auch als ein selektiv auf präsynaptische dopaminerge Rezeptoren wirkender Agonist aufgefaßt (z.B. Andén et al., Acta pharmacol, et Toxicol., 52 , 51-56 (1983) und J. Neural Transmission 57 , 129-137 (1983)).

Aus der EP-Al 192 098 ist bekannt, daß BHT 920 an postsynaptischen dopaminergen Strukturen des Gehirns ebenfalls eine agonistische Wirkung entfaltet, allerdings nur unter der Voraussetzung eines Dopaminmangels infolge Denervation oder Degeneration. Diese Entdeckung zeichnet die Verbindung im besonderen Maße zur Behandlung der Parkinsonschen Erkrankung bzw. des Parkinsonismus aus.

In der deutschen Patentanmeldung Nr. 34 47 075.1 werden Tetrahydrobenzthiazole der allgemeinen Formel

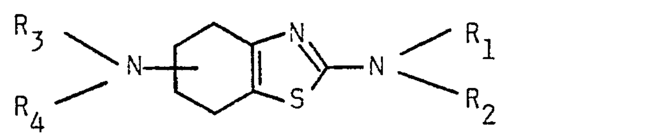

, (I)

deren Enantiomere und deren Säureadditionssalze, insbesondere deren physiologisch verträgliche Säureadditionssalze mit anorganischen oder organischen Säuren, und Verfahren zu ihrer Herstellung, beschrieben.

Bedeutet in der obigen allgemeinen Formel I einer der Reste $R_1$ oder $R_3$ oder beide der Reste $R_1$ und $R_3$ einen Acylrest, so stellen diese Verbindungen der allgemeinen Formel I wertvolle Zwischenprodukte zur Herstellung der übrigen Verbindungen der allgemeinen Formel I dar, welche wertvolle pharmakologische Eigenschaften aufweisen, insbesondere eine Wirkung auf das Zentralnervensystem und/oder den Kreislauf.

Gegenstand der vorliegenden Erfindung ist die Verwendung von Dopamin-Autorezeptor-Agonisten, insbesondere von 6-Allyl-2-amino-5,6,7,8-tetrahydro-4H-thiazolo[4,5-d]-azepin (BHT 920) und (S)-2-Amino-4,5,6,7-tetrahydro-6-n-propylamino-benzothiazol (SND 919) und ihren pharmazeutisch annehmbaren Säureadditionssalzen für die Herstellung von Medikamenten für die Behandlung von Drogenabhängigkeit. Dabei werden die Dopamin-Autorezeptor-Agonisten über längere Zeit (Wochen bis Monate ab dem Drogenentzug) verabreicht. Bei einem Rückfall des Patienten in den Drogenkonsum wird die psychische Abhängigkeit des Patienten von der Droge durchbrochen, da die vom Drogenkonsum erwartete euphorische Stimmung (Reward) ausbleibt.

Einige Verbindungen, die Dopamin-Autorezeptor-Agonisten sind, insbesondere BHT 920 und (S)-2-Amino-4,5,6,7-tetrahydro-6-n-propylamino-benzothiazol, sind aus den oben beschriebenen Patenten und Patentanmeldungen bekannt.

Zur Behandlung von Drogenabhängigkeit lassen sich die Verbindungen und ihre geeigneten Säureadditionssalze in die üblichen galenischen Zübereitungsformen für orale, parenterale, rektale oder transdermale Anwendung einarbeiten.

Die orale Einzeldosis beträgt am Menschen normalerweise zwischen 2,5 µg und 350 µg, vorzugsweise zwischen 100 µg und 250 µg; bei Mehrfachapplikation (z.B. 3 x täglich) beträgt die Tagesdosis bevorzugt zwischen 15 und 900 µg, vorzugsweise zwischen 30 und 750 µg. Die parenteralen und rektalen Dosen können in gleichem Bereich wie die oral anzuwendenden Substanzmengen liegen.

Im folgenden werden einige in der Literatur gebräuchliche Ausdrücke erläutert:

Reward (inner reward): "innere Belohnung", euphorische Empfindung, Lustgewinn, durch Drogenkonsum ausgelöst.

Reinforcement (positive reinforcement): "positive Verstärkung"; im Tierversuch durch Belohnung ausgelöste Wiederholung einer erlernten Handlung.

Drug Craving: Bedürfnis bzw. starkes Verlangen nach einer Droge, z.B. wie es meist nach dem Entzug einer Droge auftritt.

Durch Tierversuch ist gezeigt worden, daß Medikamente, die ein Mißbrauchspotential haben, wie z.B. Kokain, Opiate und Alkohol, in verschiedenen Zonen des Gehirns die Freisetzung von Dopamin (DA) herbeiführen (5, 13, 24). Weiterhin (28) wurde gezeigt, daß intracraniale elektrische Stimulation von DA-reichen Gehirnzonen hohe Raten von Selbststimulation bewirken. Aus diesen Befunden ist geschlossen worden, daß die DA-Freisetzung einen Reward bewirkt und so zu einer positiven Verstärkung (positive reinforcement) führt. Damit wäre die Basis für eine psychische Abhängigkeit gegeben.

Im Gegensatz dazu führt die chronische Administration von z.B. psychomotorischen Stimulantien und Opiaten zu DA-Verarmung im Gehirn und demzufolge zu Supersensitivität von postsynaptischen DA-Rezeptoren; durch diese Beobachtung kann die Entwicklung von Toleranz und Drug Craving nach dem Entzug erklärt werden (1, 5, 14, 16, 17, 27).

Es ist ferner über klinische Untersuchungen berichtet worden (9, 20, 8, 11, 15, 7, 10, 27, 23), in denen DA-Agonisten (Amantadine, Bromocriptine, L-Dopa) beim Drogenentzug (insbesondere Kokain) verwendet wurden. In den meisten Fällen wird in diesen Berichten darauf hingewiesen, daß die Verabreichung der DA-Agonisten eine Verminderung des Drug Cravings bewirkt. Diesem positiven Ergebnis steht jedoch entgegen, daß DA-Agonisten ein Mißbrauchspotential haben (19, 21, 25). Dieses Mißbrauchspotential ist auch für die obengenannten DA-Agonisten Amantadine, Bromocriptine und L-Dopa verständlich, da es sich bei diesen Mitteln um postsynaptische DA-Agonisten handelt, die durch ihre DA-artige Wirkung einen Reward bewirken.

Die genannten klinischen Untersuchungen wurden bei chronisch Süchtigen gemacht, bei denen DA-Mangel und Supersensitivität von postsynaptischen Rezeptoren vorliegt. Ist die Supersensitivität auf einen Teil der postsynaptischen Rezeptoren beschränkt, so kann durch die DA-artige Wirkung der Substanzen auf das normale System das Mißbrauchspotential zum Tragen kommen. Das Mißbrauchspotential kann auch zum Tragen kommen, wenn einige Zeit nach dem Entzug Desensibilisierung der postsynaptischen Rezeptoren eintritt.

Aufgrund der genannten Befunde (Mißbrauchspotential) muß man zu dem Schluß kommen, daß Dopamin-Rezeptor-Agonisten für die Behandlung der psychischen Abhängigkeit bei weiterbestehendem Drogenmißbrauch nicht und für die Behandlung des Drug Cravings beim Entzug nur sehr bedingt geeignet

sind.

Es wurde nun festgestellt, daß im Gegensatz zu den Dopamin-Rezeptor-Agonisten die DA-Autorezeptor-Agonisten mit dem Wirkungsprofil von beispielsweise BHT 920 und (S)-2-Amino-4,5,6,7-tetrahydro-6-n-propylamino-benzo-thiazol (SND 919) für die Behandlung von Drogenmißbrauch sehr gut geeignet sind.

In den US-Patentschriften 4,426,386 und 4,612,316 werden substituierte Phenylpiperidine beziehungsweise tricyclische Amine als Dopamin-Autorezeptor-Agonisten beschrieben. Die Patentschriften enthalten einen Hinweis auf die Verwendung dieser Verbindungen bei Drogenmißbrauch. Von den in den genannten US-Patentschriften behandelten Dopamin-Autorezeptor-Agonisten unterscheiden sich die erfindungsgemäß verwendeten Dopamin-Autorezeptor-Agonisten durch ihr spezielles Wirkungsprofil. Dieses weiter unten genauer beschriebene Wirkungsprofil bedingt, daß sie besonders gut für die Behandlung aller Komponenten des Drogenmißbrauchs geeignet sind.

DA-Autorezeptor-Agonisten verringern die Freisetzung und Synthese von Dopamin aus Zellen des mesolimbischen und nigro-striatalen Systems (29). Diese Verringerung wirkt der durch Drogen (z.B. Kokain) bewirkten Steigerung der DA-Freisetzung entgegen. Wenn bei der Behandlung der Drogenabhängigkeit der Patient einen solchen DA-Autorezeptor-Agonisten vorzugsweise regelmäßig anwendet, wird bei einem Rückfall in den Drogenmißbrauch der erwartete Reward nicht erzielt, da es nicht zu einer ausreichenden Steigerung der DA-Freisetzung kommt. Das Bedürfnis nach einer Wiederholung des Lustgewinns wird somit nicht wieder geweckt und die psychische Abhängigkeit wird durchbrochen. So kann das Absetzen der Droge erleichtert und die Gefahr eines Rückfalls nach dem Drogenentzug verringert werden. Damit ist erstmals die Möglichkeit gegeben, die psychische Abhängigkeit bei Drogenmißbrauch zu behandeln.

In ausführlichen Tierversuchen ist nunmehr festgestellt worden, daß diese DA-Autorezeptor-Agonisten (z.B. BHT 920 und SND 919) kein Mißbrauchspotential haben. Das ist für die Therapie von großer Bedeutung.

Eine weitere Wirkung der bevorzugten DA-Autorezeptor-Agonisten unterstützt die oben beschriebene Therapie. Die Verbindungen stimulieren anders als die oben beschriebenen postsynaptischen DA-Agonisten nicht allgemein alle postsynaptischen DA-Rezeptoren und damit die dopaminerg innervierten Gehirnzellen, sondern nur die nach chronischem Drogenmißbrauch supersensitiven DA-Rezeptoren. Es wird in (30) darauf in Zusammenhang mit der Behandlung der Parkinson'schen Krankheit hingewiesen. Diese Stimulierung ist bei Patienten mit einem längeren Drogenmißbrauch von Bedeutung, da bei diesen Patienten durch eine DA-Verarmung der Gehirnzellen ein Teil der DA-Rezeptoren überempfindlich sind, was als Ursache des Drug Cravings im Entzug gilt.

Wie bereits erwähnt worden ist, wirken diese DA-Autorezeptor-Agonisten nur auf die (z.B. durch chronischen Drogenmißbrauch) supersensitivierten Rezeptoren und lindern so das Drug Craving. Sie wirken aber nicht auf das normale System und auch nicht auf Rezeptoren, die nach einiger Zeit der Behandlung desensibilisiert sind. Die DA-Autorezeptor-Agonisten weisen somit kein Mißbrauchpotential auf.

Ein weiterer Vorteil der Anwendung mancher DA-Autorezeptor-Agonisten, z.B. BHT 920 liegt darin, daß diese durch ihre zentralen alpha-agonistischen Eigenschaften die physischen Entzugserscheinungen erleichtern.

Durch die folgenden Tierversuche können die obenbeschriebenen Wirkungen belegt werden.

Test 1

Der Versuch wurde an 3 Affen ausgeführt, die Erfahrung mit intravenöser Selbstadministration von Natriummethohexital und Kochsalzlösung haben. Sie hatten die Möglichkeit, Infusionen des Medikamentes oder der Kochsalz lösung zu erhalten und darauf zu reagieren im Verlauf von täglich 2 Sitzungen von je 130 Minuten Dauer. Zu Beginn jeder Sitzung wurde in jedem Affenkäfig ein rotes Licht eingeschaltet, das über einem der beiden Hebel angebracht war. Sobald das Licht eingeschaltet worden war, führten 10 Reaktionen auf den entsprechenden Hebel zu einer 5 Sekunden dauernden (1 ml) intravenösen Infusion der Lösung des Medikaments oder des Kochsalzes. Jeder Infusion folgte eine 10 Sekunden dauernde Unterbrechung; während der Infusion und der Unterbrechung verlöschte das rote Licht. Während der Infusionen wurde ein zentral angeordnetes grünes Licht eingeschaltet. Nach jeder Unterbrechung wurde das rote Licht wieder eingeschaltet. Für jede Sitzung waren maximal 200 Infusionen vorgesehen, aber keiner der Affen verabreichte sich die maximale Anzahl von Infusionen.

Den Affen wurde während je ungefähr der Hälfte der Dauer der Basissitzungen Natriummethohexital (0,1 mg/kg/Injektion) und Kochsalzlösung angeboten.

Bevor die Wirkung der Testsubstanz überprüft wurde zeigte jeder Affe eine klar und beständig unterschiedliche Reaktion auf Natriummethohexital und Kochsalz.

In ähnlicher Weise verhielten sich die Benzodiazepine Diazepam und Midazolam in dieser Versuchsanordnung, so daß der Versuch für Barbituraten und Benzodiazepinen ähnliche Suchtpotentiale aussagekräftig ist.

Vier Dosen der Testsubstanz BHT 920 wurden untersucht; jede Dosis wurde zweimal in jedem der Affen untersucht. Die Anzahl der genommenen Injektionen der Testsubstanz war nicht höher als die Anzahl der genommenen Injektionen von Kochsalzlösung und wesentlich niedriger als die der genommenen Methohexitalinjektionen. Der Einfluß der Größe der Dosis ist unwesentlich. Diese Ergebnisse zeigen, daß die Testsubstanz bei den angewendeten Dosen kein Reinforcement zeigt.

Bei der Durchführung der Untersuchung wurden folgende durchschnittlichen Werte ermittelt.

In der Sitzung, die unmittelbar vor der Sitzung mit der Testsubstanz durchgeführt wurde, ergaben sich bei 3 Tieren zwischen 120 und 140 Selbstinjektionen von 0,1 mg/kg/Injektion Methohexital und ≦ 20 Selbstinjektionen von Kochsalzlösung. In den Sitzungen, in denen die Testsubstanz BHT 920 verwendet wurde, ergaben sich folgende Werte:

| mg BHT 920/kg/Injektion | Anzahl der Selbstinjektionen |
|---|---|
| 0,0001 | 21 |
| 0,001 | 19 |
| 0,05 | 18 |
| 0,01 | 10 |

Daraus kann geschlossen werden, daß BHT 920 kein Barbituraten und Benzodiazepinen ähnliches Suchtpotential aufweist.

Test 2

Die Testsubstanz BHT 920 wurde in einer dem Test 1 analogen Versuchsanordnung untersucht, in der die Affen intravenöse Infusionen von Kokain erhalten. In dieser Versuchsanordnung wurden 4 Dosen von Kokain oder Testsubstanz in jeder der 130 Minuten dauernden Sitzung den Affen zugänglich gemacht. Jede Dosis war während 25 Minuten zugänglich oder so lange bis 20 Infusionen genommen worden waren. Der Zeitplan der Verabreichung der Medikamente war ein festgelegtes Verhältnis von 30 zu 45 Sekunden. Zwischen der Verabreichung der einzelnen Dosen war eine Unterbrechung von 10 Minuten. Bei jedem der 3 Affen hielt Kokain eine dosenabhängige Erhöhung der Reaktionshäufigkeit aufrecht. BHT 920 führte nur zu einer Reaktionshäufigkeit, die in dem Bereich lag, der auch bei Kochsalzlösung beobachtet wurde. Eine Dosisabhängigkeit in irgendeiner Weise war nicht festzustellen.

Die Dosen von BHT 920 waren 0,001, 0,003 und 0,01 mg/kg/Injektion.

Daraus kann geschlossen werden, daß BHT 920 kein Kokain ähnliches Suchtpotential aufweist.

Test 3

Nach Durchführung des Tests Nr. 2 wurde in der Sitzung, die der Verabreichung von BHT 920 folgte, beobachtet, daß bei 2 der 3 Versuchstiere die durch Kokain verursachte Reaktionshäufigkeit wesentlich unter der in Test 2 für Kokain beobachtete Reaktionshäufigkeit lag. In weiteren analogen Versuchen (siehe beiliegende graphische Darstellungen für Affe 1, Affe 2 und Affe 3) wurde den drogenabhängigen Affen BHT 920 in 2 Dosen (0,01 und 0,1 mg/kg/Injektion) gleichzeitig zu steigenden Dosen von Kokain (2 Tiere) beziehungsweise Alfentanil (1 Tier) angeboten. Die zusätzliche Gabe von BHT 920 in der niedrigeren Dosierung führte bei allen Tieren zu einer deutlichen Abnahme der Kokain -beziehungsweise Alfentanil -Selbstadministrationsrate. Die hohe Dosierung ließ bei allen Tieren die Rate auf Null sinken. Auch sehr hohe Dosen der suchterzeugenden Droge konnten diese Wirkung von BHT 920 nicht aufheben. Aufgrund von Verhaltensbeobachtungen während der oben beschriebenen Tests kann gesagt werden, daß die hemmende Wirkung von BHT 920 nicht durch eine allgemeine Sedation erklärt werden kann. Daraus kann geschlossen werden, daß die Verabreichung von BHT 920 das Bedürfnis nach Kokain mindert und die psychische Abhängigkeit von der Droge durchbricht.

Die Vorteile der erfindungsgemäßen Verwendung von BHT 920 können wie folgt zusammengefaßt

werden: Da BHT 920 selbst keine Sucht auslöst, kann dieses Mittel über lange Zeit (Monate oder Jahre) verabreicht werden, ohne daß die bekannte Gefahr besteht, die erste Sucht durch eine neue (eventuell weniger schädliche) Sucht zu ersetzen.

Im Entzug (bei stufenweisem oder vollständigem Absetzen der Droge) leidet der Patient unter Folgen des eintretenden Dopaminmangels im Gehirn (z.B. Depression) und unter körperlichen Entzugserscheinungen wie Störungen des Herz-Kreislaufsystems, Störungen von Drüsenfunktionen, Zittern etc.. Regelmäßige Verabreichung von BHT 920 lindert oder belebt die körperlichen Entzugserscheinungen durch die $\alpha_2$-agonistische Wirkung. In der vorliegenden Situation des Dopaminmangels bewirkt BHT 920 außerdem durch seine postsynaptische Dopamin $D_2$-agonistische Wirkung eine Anhebung des Dopaminangebotes im Gehirn auf ein dem gesunden System entsprechendes Niveau. Dadurch wird eine Ursache der Depression des Patienten behoben, ohne euphorische Empfindungen (Reward) auszulösen. Patienten ohne Behandlung erleben durch die Drogeneinnahme ein Reward, wodurch die psychische Abhängigkeit von der Droge verstärkt wird. Bei Patienten, die zwar entzugswillig sind, trotzdem aber (noch oder wieder) Drogen einnehmen, führt die regelmäßige Einnahme von BHT 920 zu einer Verminderung der psychischen Abhängigkeit. Das ist darauf zurückzuführen, daß die Dopamin-Autorezeptor-agonistische Wirkung des BHT 920 die Freisetzung von zusätzlichem Dopamin verhindert. Dadurch erlebt der Patient bei regelmäßiger Einnahme von BHT 920 trotz der Drogenadministration kein Reward. Die psychische Abhängigkeit des Patienten von der Droge wird dadurch durchbrochen.

Aus dieser Zusammenstellung wird deutlich, daß die erfindungsgemäße Verwendung von BHT 920 gegenüber herkömmlichen Behandlungsmethoden im Drogenentzug wesentliche Vorteile aufweist.

SND 919 ist ein Wirkstoff, der in seinem Wirkungsprofil dem BHT 920 analog ist. Es ist bekannt, daß SND 919 ein Dopamin-Autorezeptor-Agonist mit postsynaptischer $D_2$-agonistischer Wirkung unter den Bedingungen einer Dopamin-Depletion ist, der zusätzlich zentrale $\alpha_2$-agonistische Wirkung hat.) SND 919 ist erfindungsgemäß wie BHT 920 im Drogenentzug zu verwenden. Dasselbe gilt für Dopamin-Autorezeptor-Agonisten mit analogem Wirkungsprofil.

## Literatur:

(1) Ahtee L; Attila P; Lauhakangas V; Solkinen A; Sipilae J, J Neural Transm, 68, 63-78 (1987)
(5) Dackis CA; Gold MS; Davies RK; Sweeney DR, Int J Psychiatry Med, 15, 125-35, (1985-86)
(7) Dackis CA; Gold MS; Sweeney DR; Byron LP Jr; Climko R, Psychiatry Res, 20, 261-4, (1987)
(8) Extein IL; Gross DA; Gold MS, Am J Psychiatry, 146, 403, (1989)
(9) Gawin FH; Morgan C; Kosten TR; Kleber HD, Psychopharmacology, 97, 402-3, (1989)
(10) Giannini AJ; Billett W, J Clin Pharmacol, 27, 549-54, (1987)
(11) Handelsman L; Chordia PL; Escovar IL; Marion IJ; Lowinson JH, Am J Psychiatry, 145, 533, (1988)
(13) Kiianmaa K; Tabakoff B, Pharmacol Biochem Behav, 18, 383-8, (1983)
(14) Kolta MG; Shreve P; De Souza V; Uretsky NJ, Neuropharmacology, 24, 823-9, (1985)
(15) Kosten TR; Schumann B; Wright D, Am J Psychiatry, 145, 381-2, (1988)
(16) Kovacs GL; Horvath Z; Sarnyai Z; Faludi M; Telegdy G, Neuropharmacology, 24, 413-9, (1985)
(17) Kovacs GL; Telegdy G; Hodi K, Pharmacol Biochem Behav, 21, 345-8, (1984)
(19) Nausieda PA, Clin Neuropharmacol, 8, 318-27, (1985)
(20) Pike RF, Postgrad Med, 85, 115-6, (1989)
(21) Priebe S, Pharmacopsychiatry, 17, 109-10, (1984)
(23) Rosen H; Flemenbaum A; Slater VL, Am J Psychiatry, 143, 1493, (1986)
(24) Sonsalla PK; Gibb JW; Hanson GR, J Pharmacol and Exp Ther, 238, 932-37, (1986)
(25) Tack E; De Cuypere G; Jannes C; Remouchamps A, Acta Psychiatr Scand, 78, 356-60, (1988)
(27) Tennant FS Jr; Sagherian AA, Arch Int Med, 147, 109-12, (1987)
(28) Wauquier A, In: Brain-stimulation Reward (A. Wauquier, E.T. Rolls, eds.) North-Holland Publ. Company, Amsterdam 1976
(29) Andén N.E. et al., Naunyn-Schmiedeb. Arch. Pharmacol., 321, 100 (1982)
(30) Hinzen et al., Eur. J. Pharmacol. 131, 75-86, (1986)

Die Anwendung der DA-Autorezeptor-Agonisten kann in den für diese Verbindungen an sich bekannten Zübereitungen erfolgen. Die Mittel werden vorzugsweise regelmäßig und unter Umständen über lange Zeit verabreicht.

Beispiel I

Dragéekern

| Zusammensetzung: | |
|---|---|
| 1 Dragéekern enthält: | |
| B-HT 920 | 50 µg |
| Milchzucker | 38,45 mg |
| Maisstärke | 10,0 mg |
| Gelatine | 1,0 mg |
| Magnesiumstearat | 0,5 mg |
| | 50,0 mg |

Herstellungsverfahren:

Die Mischung der Wirksubstanz mit Milchzucker und Maisstärke wird mit einer 10 %igen wäßrigen Gelatinelösung durch Sieb 1 mm granuliert, bei 40° C getrocknet und nochmals durch obiges Sieb gerieben. Das so erhaltene Granulat wird mit Magnesiumstearat gemischt und zu Drageéekernen verpreßt. Die Herstellung muß in abgedunkelten Räumen vorgenommen werden.
Kerngewicht: 50 mg
Stempel: 5 mm, gewölbt

Die so erhaltenen Dragéekerne werden nach bekanntem Verfahren mit einer Hülle überzogen, die im wesentlichen aus Zucker und Talkum besteht. Die fertigen Dragées werden mit Hilfe von Bienenwachs poliert. Dragéegewicht: 100 mg

Beispiel II

Suppositorien

| 1 Zäpfchen enthält: | |
|---|---|
| B-HT 920 | 100,0 µg |
| Zäpfchenmasse (z.B. Witepsol W 45) | 1690,0 mg |

Herstellungsverfahren:

Die feingepulverte Substanz wird mit Hilfe eines Eintauchhomogenisators in die geschmolzene und auf 40° C abgekühlte Zäpfchenmasse eingerührt. Die Masse wird bei 35° C in leicht vorgekühlte Formen ausgegossen.

Beispiel III

Ampullen mit 200 µg B-HT 920

| 1 Ampulle enthält: | |
|---|---|
| B-HT 920 | 200 µg |
| Zitronensäure | 7,0 mg |
| Natriumphosphat sek. • 2 $H_2O$ | 3,0 mg |
| Natriumpyrosulfit | 1,0 mg |
| Dest. Wasser | ad 1,0 ml |

Herstellungsverfahren:

In ausgekochtem und unter $CO_2$-Begasung abgekühltem Wasser werden nacheinander die Puffersubstanzen, die Wirksubstanz sowie Natriumpyrosulfit gelöst. Man füllt mit abgekochtem Wasser auf das gegebene Volumen auf und filtriert pyrogenfrei.
Abfüllung: in braune Ampullen unter Schutzbegasung
Sterilisation: 20 Minuten bei 120° C

Die Herstellung und Abfüllung der Ampullenlösung muß in abgedunkelten Räumen vorgenommen werden.

Beispiel IV

Dragées mit 1 mg B-HT 920

| 1 Dragéekern enthält: | |
|---|---|
| B-HT 920 | 100 µg |
| Milchzucker | 36,0 mg |
| Maisstärke | 12,4 mg |
| Gelatine | 1,0 mg |
| Magnesiumstearat | 0,5 mg |
| | 50,0 mg |

Herstellungsverfahren:

Analog Beispiel I.

Kerngewicht: 50 mg
Stempel: 5 mm, gewölbt
Dragéegewicht: 100 mg

Beispiel V

Dragées mit 0,2 mg B-HT 920

| 1 Dragéekern enthält: | |
|---|---|
| B-HT 920 | 0,2 mg |
| Digoxin | 0,25 mg |
| Milchzucker | 66,55 mg |
| Kartoffelstärke | 25,0 mg |
| Polyvinylpyrrolidon | 2,0 mg |
| Magnesiumstearat | 1,0 mg |
| | 120,0 mg |

Herstellungsverfahren:

Die intensive Mischung der Wirksubstanzen mit Milchzucker und Kartoffelstärke wird mit einer 10 %igen Lösung des Polyvinylpyrrolidons in Äthanol durch Sieb 1,5 mm granuliert, bei 40 °C getrocknet und nochmals durch Sieb 1,0 mm gerieben. Das so erhaltene Granulat wird mit Magnesiumstearat gemischt und zu Dragéekernen verpreßt.
Kerngewicht: 120 mg
Stempel: 7 mm, gewölbt

Die so hergestellten Dragéekerne werden nach bekanntem Verfahren mit einer Hülle überzogen, die im wesentlichen aus Zucker und Talkum besteht. Die fertigen Dragées werden mit Hilfe von Bienenwachs poliert.
Dragéegewicht: 200 mg.

Beispiel VI

Gelatine-Steckkapseln mit 300 µg B-HT 920

| 1 Kapsel enthält: | |
|---|---|
| B-HT 920 | 0,3 mg |
| Codeinphosphat | 10,0 mg |
| Weinsäure | 3,0 mg |
| Maisstärke | 85,7 mg |
| | 100,0 mg |

Herstellungsverfahren:

Die Substanzen werden intensiv gemischt und in Opak-Kapseln geeigneter Größe abgefüllt.
Kapselfüllung: 100 mg

Die Herstellung von Mitteln für die transdermale Verabreichung der Substanzen ist z.B. EP-Al-227 988 zu entnehmen.

Beispiel VII

Dragéekern mit 5 mg 2-Amino-6-n-propylamino-4,5,6,7-tetrahydro-benzthiazol-dihydrochlorid

| Zusammensetzung: | |
|---|---|
| 1 Dragéekern enthält: | |
| Wirksubstanz | 5,0 mg |
| Milchzucker | 33,5 mg |
| Maisstärke | 10,0 mg |
| Gelatine | 1,0 mg |
| Magnesiumstearat | 0,5 mg |
| | 50,0 mg |

Herstellungsverfahren

Die Mischung der Wirksubstanz mit Milchzucker und Maisstärke wird mit einer 10%igen wässrigen Gelatinelösung durch ein Sieb von 1 mm Maschenweite granuliert, bei 40 °C getrocknet und nochmals durch obiges Sieb gerieben. Das so erhaltene Granulat wird mit Magnesiumstearat gemischt und zu Dragéekernen verpreßt. Die Herstellung muß in abgedunkelten Räumen vorgenommen werden.
Kerngewicht: 50 mg
Stempel: 5 mm, gewölbt

Die so erhaltenen Dragéekerne werden nach bekanntem Verfahren mit einer Hülle überzogen, die im wesentlichen aus Zucker und Talkum besteht. Die fertigen Dragées werden mit Hilfe von Bienenwachs poliert.
Dragéegewicht: 100 mg

Beispiel VIII

Suppositorien mit 10 mg 2-Amino-6-n-propylamino-4,5,6,7-tetrahydro-benzthiazol-dihydrochlorid

| 1 Zäpfchen enthält: | |
|---|---|
| Wirksubstanz | 10,0 mg |
| Zäpfchenmassen (z.B. Witepsol W 45) | 1 690,0 mg |
| | 1 700.0 mg |

Herstellungsverfahren:

Die feingepulverte Substanz wird mit Hilfe eines Eintauchhomogenisators in die geschmolzene und auf 40 °C abgekühlte Zäpfchenmasse eingerührt. Die Masse wird bei 35 °C in leicht vorgekühlte Formen ausgegossen.
Zäpfchengewicht: 1,7 g

Beispiel IX

Ampullen mit 5 mg 2-Amino-6-n-propylamino-4,5,6,7-tetrahydro-benzthiazol-dihydrochlorid

| 1 Ampulle enthält: | |
|---|---|
| Wirksubstanz | 5,0 mg |
| Zitronensäure | 7,0 mg |
| Natriumphosphat sek. x 2 $H_2O$ | 3,0 mg |
| Natriumpyrosulfit | 1,0 mg |
| Dest. Wasser | ad 1,0 ml |

Herstellungsverfahren:

In ausgekochtem und unter $CO_2$-Begasung abgekühltem Wasser werden nacheinander die Puffersubstanzen, die Wirksubstanz sowie Natriumpyrosulfit gelöst. Man füllt mit abgekochtem Wasser auf das gegebene Volumen auf und filtriert pyrogenfrei.
Abfüllung: in braune Ampullen unter Schutzbegasung
Sterilisation: 20 Minuten bei 120°C

Die Herstellung und Abfüllung der Ampullenlösung muß in abgedunkelten Räumen vorgenommen werden.

Beispiel X

Dragées mit 1 mg 2-Amino-6-n-propylamino-4,5,6,7-tetrahydro-benzthiazol-dihydrochlorid

| 1 Dragéekern enthält: | |
|---|---|
| Wirksubstanz | 1,0 mg |
| Milchzucker | 35,5 mg |
| Maisstärke | 12,0 mg |
| Gelatine | 1,0 mg |
| Magnesiumstearat | 0,5 mg |
| | 50,0 mg |

Herstellungsverfahren:

Analog Beispiels I.

Kerngewicht: 50 mg
Stempel: 5 mm, gewölbt
Dragéegewicht: 100 mg

## Ansprüche

1. Verwendung von Dopamin-Autorezeptor-Agonisten für die Herstellung von Medikamenten für die Behandlung von Drogenabhängigkeit.
2. Verwendung nach Anspruch 1, dadurch gekennzeichnet, daß die Behandlung während des Entzuges und über einen darüber hinausgehenden Zeitraum erfolgt und der Durchbrechung der psychischen Abhängigkeit des Patienten von der Droge dient.
3. Verwendung nach Anspruch 1 oder 2, dadurch gekennzeichnet, daß der Agonist neben seiner Dopamin-Autorezeptor-agonistischen Wirkung auch postsynaptische Dopamin $D_2$-agonistische Wirkung unter der

Bedingung einer Dopamin-Depletion aufweist.

4. Verwendung nach Anspruch 3, dadurch gekennzeichnet, daß der Agonist zusätzlich zentrale $\alpha_2$-agonistische Wirkung aufweist.

5. Verwendung nach einem der Ansprüche 1 bis 4, dadurch gekennzeichnet, daß der Dopamin-Autorezeptor-Agonist 6-Allyl-2-amino-5,6,7,8-tetrahydro-4H-thiazolo[4,5-d]-azepin (BHT 920) oder ein pharmazeutisch annehmbares Säureadditionssalz davon ist.

6. Verwendung nach einem der Ansprüche 1 bis 4, dadurch gekennzeichnet, daß der Dopamin-Autorezeptor-Agonist (S)-2-Amino-4,5,6,7-tetrahydro-6-n-propylamino-benzothiazol oder ein pharmazeutisch annehmbares Säureadditionssalz davon ist.

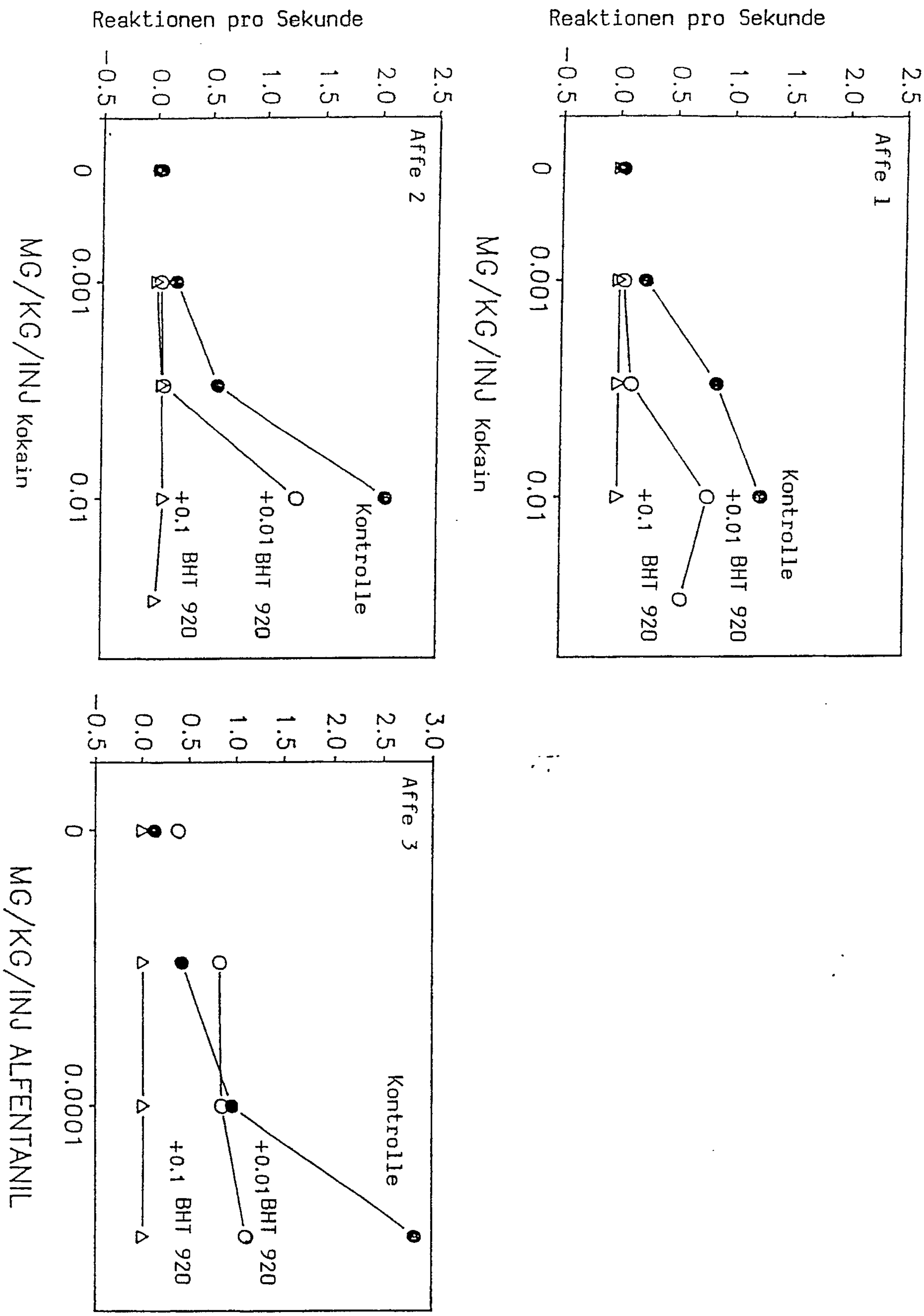
Reaktionen pro Sekunde
Affe 1
Kontrolle
+0.01 BHT 920
+0.1 BHT 920
MG/KG/INJ Kokain
Reaktionen pro Sekunde
Affe 2
Kontrolle
+0.01 BHT 920
+0.1 BHT 920
MG/KG/INJ Kokain
Affe 3
Kontrolle
+0.01 BHT 920
+0.1 BHT 920
MG/KG/INJ ALFENTANIL